(19) Europäisches Patentamt
European Patent Office
Office européen des brevets

(11) **EP 4 345 456 B1**

(12) **EUROPEAN PATENT SPECIFICATION**

(45) Date of publication and mention
of the grant of the patent:
**12.11.2025 Bulletin 2025/46**

(51) International Patent Classification (IPC):
**G01N 33/74** *(2006.01)* **G01N 33/68** *(2006.01)*

(52) Cooperative Patent Classification (CPC):
**G01N 33/743**; G01N 2800/367

(21) Application number: **23199762.8**

(22) Date of filing: **26.09.2023**

(54) **METHOD FOR ASSESSING OOCYTES QUALITY**

VERFAHREN ZUR BEURTEILUNG DER OOZYTENQUALITÄT

PROCÉDÉ D'ÉVALUATION DE LA QUALITÉ D'OVOCYTES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **28.09.2022 IT 202200019977**

(43) Date of publication of application:
**03.04.2024 Bulletin 2024/14**

(73) Proprietor: **Global Life S.r.l.**
**80121 Napoli (NA) (IT)**

(72) Inventors:
• **Dale, Brian**
**80121 Napoli (NA) (IT)**
• **Abrescia, Paolo**
**80121 Napoli (NA) (IT)**

(74) Representative: **Pace Napoleone, Maria et al**
**De Simone & Partners S.r.l.**
**Via Giulio Caccini, 1**
**00198 Roma (IT)**

(56) References cited:
• **VIHMA VEERA ET AL: "Quantitative Determination of Estradiol Fatty Acid Esters in Human Pregnancy Serum and Ovarian Follicular Fluid", vol. 47, no. 7, 1 July 2001 (2001-07-01), US, pages 1256 - 1262, XP093016511, ISSN: 0009-9147, Retrieved from the Internet <URL:https://academic.oup.com/clinchem/article-pdf/47/7/1256/32724794/clinchem1256.pdf> DOI: 10.1093/clinchem/47.7.1256**

• **CIGLIANO L ET AL: "Estradiol esterification in the human preovulatory follicle", STEROIDS, ELSEVIER SCIENCE PUBLISHERS, NEW YORK, NY, US, vol. 66, no. 12, 1 December 2001 (2001-12-01), pages 889 - 896, XP004322401, ISSN: 0039-128X, DOI: 10.1016/S0039-128X(01)00124-6**

• **CHEN FANG ET AL: "Follicular fluid biomarkers for human in vitro fertilization outcome: Proof of principle", vol. 14, no. 1, 1 December 2016 (2016-12-01), XP093016854, Retrieved from the Internet <URL:https://www.ncbi.nlm.nih.gov/pmc/articles/PMC5109724/pdf/12953_2016_Article_106.pdf> DOI: 10.1186/s12953-016-0106-9**

• **ALBERTO REVELLI ET AL: "Follicular fluid content and oocyte quality: from single biochemical markers to metabolomics", REPRODUCTIVE BIOLOGY AND ENDOCRINOLOGY, vol. 7, no. 1, 4 May 2009 (2009-05-04), XP055716339, DOI: 10.1186/1477-7827-7-40**

EP 4 345 456 B1

## Description

## FIELD OF THE INVENTION

The present invention is directed to a method for the evaluation of oocytes quality and/or of the response to treatments to improve the development of follicles with high fertilization potential. In women with normal cycles, plasma or serum analysis of the $E_{EST}$ level provides information on oocyte quality and the probability of successful embryonic development following in vitro fertilization of the oocyte. Analysis of the biomarker $E_{EST}$, in plasma or serum of patients diagnosed with diseases associated with infertility, can be done repeatedly to monitor the progression of follicular development and oocyte maturation or the individual response to therapy.

## BACKGROUND OF THE INVENTION

Infertility is an emerging health problem that is often underestimated. Approximately 10% to 15% of couples in developed countries are infertile. About a third of infertility cases are of unknown origin and two-thirds depend with equal probability on both male and female pathologies, which can cause dysfunctions in the production of gametes suitable for fertilization and embryonic development. Apart from genetic causes, it is believed that environmental alterations (including various and different aspects such as malnutrition, air or water or food pollution, physical or mental stress, etc.) and diseases adversely affect human reproduction. The World Health Organization has recognized that infertility is a disease and that assisted reproductive technology (ART, namely IVF or ICSI) methods [https://www.sart.org/professionals-and-providers/research/publications-using-sart-cors/] are a way of treating it. The etiology of many types of infertility is currently not fully understood and there is little information on some molecular basis of the mechanisms underlying the processes that drive ovarian follicle development, oocyte maturation and embryonic development [1-5]. It is well known that ovarian follicular fluid (FF) contains factors that are very important for oocyte maturation and for the oocyte ability to form, after fertilization, an embryo able to develop [6-11]. In this context, molecules or enzymatic activities capable of counteracting the attack of reactive oxygen or nitrogen species play a key role. When, or if, antioxidant defense systems (both scavenger and enzymatic type) fail to contain or neutralize oxidizing species, a pathological condition called oxidative stress arises [7, 12-16]. In this condition lipids, carbohydrates, proteins and nucleic acids undergo structural damage that generally leads to loss of molecular or cellular functions. Some antioxidants in FF originate from Granulosa cells, while others enter the follicle from circulation through the blood-follicle barrier [7, 17,18]. In particular, the levels of ascorbic acid (vitamin C) and alpha-tocopherol (vitamin E) were monitored in FF taken from preovulatory follicles with different values of the ratio between estradiol (E), also spelled oestradiol, and progesterone (P) [19], an index of follicular development that decreases as ovulation approaches. In particular, the E/P ratio was found to be negatively correlated with the activity of the enzyme LCAT (Lecithin-Cholesterol Acyltransferase, EC 2.3.1.43), measured ex vivo. Instead, this enzymatic activity, known to be degraded by oxidants, was found in FF with positive correlation with the level of ascorbic acid or alpha-tocopherol, but with negative correlation with the level of nitrotyrosines (index of oxidative damage to proteins) [20]. It is therefore observed that the activity of LCAT, as damaged by oxidative stress and as associated with known markers of the redox state, represents a new marker of oxidative stress or ovarian follicular redox state. A large literature reports that other types of antioxidants such as, for example, glutathione or generically compounds comprising sulfhydryl groups, have also been found at elevated levels in FF from preovulatory follicles in physiological development. In addition, many publications report data on the total antioxidant capacity (i.e. relative to all molecules capable of neutralizing oxidants or limiting oxidative damage in general) in FF and claim that follicular or oocyte quality is associated with high levels of antioxidants.

The enzyme LCAT, already mentioned as a target (and therefore as a marker) of oxidative stress, plays an important role in helping to remove excess cholesterol from peripheral tissue cells, since this excess is in fact cytotoxic [21-23]. In particular, the accumulation of cholesterol in Granulosa cells can increase P biosynthesis, which can reduce the activity of the aromatase enzyme [6, 24-28] and induce follicular atresia [29]. LCAT esterifies cholesterol, so that this steroid can be transferred from the cell plasma membrane to high-density proteins (HDL), which are then transported to the circulation for hepatic cholesterol metabolism (i.e. for bile acid synthesis) and for its elimination from the body in feces [21]. The efflux of cholesterol from Granulosa cells to HDL has been previously described [30, 31] or suggested [32, 33]. The activity of LCAT is therefore necessary to prevent cytotoxicity due to excess cholesterol in peripheral tissue cells also because, under conditions of normal ovarian function (and particularly during follicular development), plasma HDLs cross the blood-follicle barrier [30, 31, 34-37] precisely to provide cholesterol to both theca and Granulosa cells as the only source of sex steroids [38]. There must be a balance between the supply and removal of cholesterol in the follicle. In this scenario, LCAT can limit the amount of cholesterol in ovarian cells, in order to prevent its accumulation on the plasma membrane, removing what exceeds the physiological need for the synthesis of sex steroids.

LCAT is known to esterify, in addition to cholesterol, other steroids, including E [39]. Estradiol esters ($E_{EST}$) are transported by HDL into the circulation and are addressed to tissues such as adipose, where they represent a deposit of E protected until the hormone is freed of the acyl chain, by means of esterases suitably stimulated, for its action [40]. Thus $E_{EST}$ represent a powerful and long-lasting reserve of E [41], with topical (especially on the ovary) and systemic effects. It is evident that the transport of $E_{EST}$, mediated by HDL, is massive, that is, much greater than that which occurs by association with specific or nonspecific protein transporters such as, for example, respectively the "sex steroid-binding proteins" or albumin. Elevated levels of $E_{EST}$ have been found in human preovulatory follicles [19]. In particular, it has been suggested that, during the progression of the preovulatory phase of follicular development, the esterification of E increases while that of cholesterol decreases.

Testing new drugs, in general, requires criteria and tools to identify promising lead compounds and to assess patient response to treatment. In this context, there are no known biomarkers of oocyte quality that unequivocally reflect the follicular redox state and, as such, have been approved by the FDA or EMA to evaluate the efficacy of a therapy intended to improve fertilization and embryonic development. The evaluation of the efficacy of therapy, especially in terms of dosage of a drug or duration of administration, requires analytical processes and reliable probes or biomarkers to observe the patient's response. There is no way, with current laboratory analysis, to support the development of new drugs for the treatment of female infertility. Likewise, there is no way to monitor the individual response to any treatment and, therefore, to modulate therapeutic interventions to patients with different pathophysiological conditions.

The success of fertilization depends on the quality of gametes. Such quality, in turn, depends on several factors that affect the process of maturation in physiological conditions or under drug treatment. In particular, it is well known that the sperm of a fertile man, when put together with some oocytes of a woman stimulated for superovulation in ART, form fully functional gametes with only part of them, while most oocytes remain unfertilized or form zygotes that do not proceed towards embryonic development. To date, oocytes with a positive fertilization outcome cannot be distinguished from oocytes with a negative fertilization outcome only according to their morphology. Known methods of selection of good quality oocytes include the morphological evaluation of the cumulus-oocyte complex (COC), such as the localization of the mitotic spindle with respect to the position of the polar body [42-45] and the metabolomic profile of the FF [8, 46-52]. These data add further evidence to the hypothesis that morphological characteristics of individual COCs are not good markers of oocyte quality [42-45, 53].

Subjective morphological parameters are still a primary criterion for selecting healthy embryos to be used for IVF and ICSI programs in ART. However, these criteria do not really predict an embryo's ability to develop. Many studies have shown that a combination of several different morphological criteria leads to a more accurate selection of the embryo to be transferred for implantation [54-58]. Morphological criteria for embryo selection are established on the day of transfer and are mainly based on early embryo division (25-27 hours after insemination), the number and size of blastomeres on the second or third day, the percentage of fragmentation and the presence of multiple nucleation in the 4 or 8 cell stage [59]. However, a recent study has shown that the selection of oocytes for insemination does not improve the success of ART compared to the transfer of all available embryos, regardless of their quality [58]. There is a need to identify viable embryos with the highest implantation potential to increase the IVF success rate, reduce the number of embryos to be transferred and lower the rate of multiple pregnancies.

Although redox homeostasis in FF is thought to contribute to oocyte health and function, very little information is currently available in the scientific literature about the positive correlation between follicular antioxidant defenses and oocyte quality or about the negative correlation between follicular oxidative alterations and oocyte quality. No biomarker has been validated to date for the evaluation of the redox status of FF, in order to predict the fertilization potential of the oocyte. In addition, to the extent that plasma contributes to redox homeostasis in the follicle, biomarkers of oxidative damage or antioxidant capacity in plasma are not used in clinical practice to predict whether healthy oocytes are released from follicles with high antioxidant defenses.

The detection of oocyte quality biomarkers and the measurement of their level in the ovarian follicle currently require FF sampling. The field expert knows that this procedure can be done safely when the follicle reaches an appropriate size, during the late preovulatory phase, and is ready for the release of the oocyte. The puncture of the follicle with a needle, the aspiration of the fluid and the collection of the oocyte from the fluid itself are in fact proven operations that allow an efficient and safe recovery of the oocyte for ART. Instead, these operations are at high risk when follicular development is not complete, because both fluid volume (or the total amount of nutrients/signals contained therein) and the integrity of the COC could be severely compromised. Therefore, at the moment, in women with normal cycles (i.e. who have not been stimulated for superovulation, but who instead undergo normal dominant follicle formation) the analysis of FF biomarkers, to evaluate important factors to promote follicular development and oocyte maturation, cannot be performed during the preovulatory phase.

When follicular development is defective, or in other ovarian dysfunctions, there is currently no effective treatment to improve symptoms and especially to induce remission of the disease (targeting initial or aggravating molecular events). Pharmaceutical approaches to treat female infertility need reliable biomarkers to assess oocyte quality and, to the extent that such quality is associated with FF redox homeostasis, antioxidant defenses or oxidative damage within the follicle. In addition, the search for lead compounds and the development of new drugs need criteria and tools to evaluate the (individual) response to therapy in terms of efficacy, dosage and duration of administration. Common and current analyses are not able to support the testing of new drugs or differentiated treatments, in terms of different dosages or different duration of administration, to patients in different pathological conditions.

Methods published in the scientific literature or patents to assess oocyte quality by measuring $E_{EST}$ levels (in FF, serum or plasma) have never been correlated with the outcome of ART. In fact, these methods provided limited information to the synthesis, release into the circulation and putative function (in terms of gradual and delayed estrogenic activity on target organs) of $E_{EST}$ and allowed only to ascertain that lipid peroxidation alters the redox state in the follicle and in the circulation. Only one publication suggests that the level of E linoleate (subject to great individual variability) could be related to the stage of follicular development [19], although the ratio E-Linoleate/E, supposed to reflect the LCAT activity in the follicular preovulatory phase, was not used to predict the oocyte fate in ART. Therefore data on the global level of the various types of esters with fatty acids (i.e. $E_{EST}$, the only reliable marker of the activity of the LCAT enzyme) and above all on the correlation between the global level of estradiol esters in the follicular fluid and the success of the oocyte in fertilization and embryonic development have never been published.

It is known that EEsT are reliably detectable in women receiving fertility treatments, but too low to be reliably detectable in normal cycling women, and EEsT has antioxidant (i.e. redox) properties; and that EEsT has powerful estrogenic (i.e. fertility enhancing) effects [60].

Are known different follicular fluid biomarkers for assessing fertility, oocyte quality, IVF outcome etc. [61].

In the course of the present invention, a new biomarker of follicular redox state has been identified that can be used, alone or in combination with biomarkers of antioxidant defenses or oxidative damage in the follicle, for diagnostic purposes in the management of female infertility. This invention therefore concerns the detection and use of the $E_{EST}$ level to monitor the health and development status of multiple follicles of the same ovary, in terms of redox homeostasis, and thus to select oocytes with high fertilization potential in ART. Another non-exclusive way of using the discovery of the biomarker is to detect its level in FF or plasma of subjects with normal cycle, as a tool for information on the redox state of the dominant preovulatory follicle and to predict the fate of the homologous oocyte in ART. The biomarker can also be used as a tool to assess the individual response of women undergoing treatments to improve the development of follicles containing oocytes with high fertilization potential. In addition, the biomarker can be used as a tool to evaluate adverse effects from drug overdose and/or hormonal treatment and to design and develop new drugs.

## SUMMARY OF THE INVENTION

The invention in its general sense is a method according to claim 1, and use of a kit to perform said method according to claim 10.

The invention concerns in part the detection and monitoring of the level of estradiol esters ($E_{EST}$) in ovarian follicular fluid (FF) and homologous plasma in women with normal cycles. A further aspect of the invention concerns the detection and monitoring of the level of $E_{EST}$ in FF obtained from each of different follicles, developing (or developed) in women treated with hormones for superovulation. Finally, this invention concerns the use of the $E_{EST}$ level in FF (or in homologous plasma or serum, in the case of women with normal cycles) to predict the fertilization of an oocyte and the subsequent embryonic development in assisted reproductive technology (ART). Since circulating estradiol (E), in free or esterified form, is derived almost exclusively from ovarian follicles and the level of $E_{EST}$ depends on the esterification process that takes place in the follicle, the measurement or monitoring of this level in plasma or serum is expected to provide information on factors that influence esterification, such as antioxidants or oxidative damage. This level in plasma or serum of women with normal cycles can be monitored to assess FF redox status and oocyte maturation. In women treated with hormonal stimulation, the level of $E_{EST}$ in FF from single follicles can be used to predict the fertilization potential of homologous oocytes and, therefore, to select the most promising oocytes to match with the spermatozoa. Infertile subjects with low levels of $E_{EST}$, in plasma or serum during the ovulatory phase of the cycle, can be treated to improve the follicular condition appropriate to oocyte maturation. These levels of $E_{EST}$ can also be used as biomarkers to monitor response to treatment and to predict the outcome of ART. The biomarkers proposed here can also be used to select or develop "lead" compounds in novel therapies for deficient or incomplete follicular development.

Therefore, it is an object of the invention an in vitro method for t the evaluation of the oocytes quality and/or of the response to treatments to improve the development of follicles with high fertilization potential in a subject, said method comprising the step of measuring the amount of esterified estradiol ($E_{EST}$) in an isolated sample of plasma or follicular fluid from said subject and comparing the same with a $E_{EST}$ control value indicative of subjects having oocytes with good fertilization potential.

Preferably the method of invention comprises the following steps:

    a. measuring in the sample of plasma or follicular fluid the amounts of total estradiol ($E_{TOT}$) and of non-esterified estradiol ($E_{FREE}$);

b. calculating the amount of $E_{EST}$ according to the following formula: $E_{EST} = E_{TOT} - E_{FREE}$

c. comparing the obtained value as in step b) with the $E_{EST}$ control value.

In a preferred form of realization of the invention, this method comprises:

a. introducing a predetermined amount of internal standard in the isolated sample of plasma or follicular fluid;

b. separating said sample into a first and a second aliquot;

c. treating said first aliquot by means of saponification;

d. treating both aliquots with organic extraction and taking the organic extract;

e. measuring the amount of $E_{FREE}$ in the organic extracts of the first and second aliquot and using the internal standard amounts for normalization of the obtained value;

f. calculating the amount of $E_{EST}$ according to the formula: $E_{EST} = E_{TOT} - E_{FREE}$, wherein the $E_{TOT}$ value corresponds to the amount of $E_{FREE}$ measured in the first aliquot and the $E_{FREE}$ value corresponds to the amount of $E_{FREE}$ measured in the second aliquot;

g. comparing the obtained $E_{EST}$ value with the $E_{EST}$ control value.

Preferably the internal standard is estradiol with isotopic labelling with deuterium (D) or with carbon-13 $^{13}C$, preferably 17β-Estradiol-2,4,16,16,17-$D_5$, 17β-Estradiol-16,16,17-$D_3$, 17B-Estradiol-2,4,16,16,17-$D_2$, 17β-Estradiol-2,3,4-$^{13}C_3$.

Preferably in the method of the invention the amount of $E_{EST}$ is expressed as percentage $E_{EST}/ E_{TOT} \times 100$.

It is a further object of the invention the method as described above wherein the i isolated sample is from an individual follicular fluid among multiple different fluids obtained from subjects with multiple follicles developed under hormonal stimulation treatment. Preferably the $E_{EST}$ control value is comprised between 20 and 30% and the $E_{EST}$ value measured in the biological sample of the individual follicular fluid greater than said control value is indicative of homologous oocyte with good fertilization potential.

An object of the invention is the method as described above in which the isolated plasma sample is obtained from subjects with a normal ovulatory cycle. Preferably in this method the $E_{EST}$ control value is comprised between 25 and 35% and the $E_{EST}$ value measured in the isolated plasma sample greater than said control value is indicative of oocyte with good fertilization potential.

It is an object of the invention the use of $E_{EST}$ as biomarker in an *in vitro* method for the evaluation of oocyte quality in a subject or of the response to treatments for improving the development of follicles with high fertilization potential, comprising the assessment of the redox state in a sample obtained from said subject, in an isolated sample of plasma or follicular fluid, as indicated above.

The invention further relates to use of a diagnostic kit for performing the methods of the invention, the diagnostic kit comprising the means for measuring the amount of $E_{EST}$ according to the method of invention and optionally control means. Control means can be used to compare the amount or the increase in the amount of the biomarker to a proper value from a control sample. The proper value may be obtained for example, with reference to known standard, either from a normal subject or from normal population.

Preferably the diagnostic kit is for use to evaluate the oocyte quality in a subject and/or the response to treatments addressed to improve the development of follicles with high fertilization potential, comprising the assessment of the redox state in a sample obtained from said subject, preferably in an isolated sample of plasma or follicular fluid as indicated above.

The kits (not claimed as such) can further comprise customary auxiliaries, such as buffers, carriers, markers, etc. and/or instructions for use.

In the method or a kit for the assessment of the redox state of follicular fluid of a subject and/or the evaluation of oocyte(s) quality in a subject, the proper value from a control sample (herein also identified as reference value) may be the value measured in a sample taken from a healthy patient or from a patient affected by a disease other than infertility. In the case of a method or a kit for monitoring the efficacy of or the response to a therapeutic treatment to improve the development of follicles with high fertilization potential in a subject, the proper value from a control sample may by a value measured in a sample taken from the same subject before initiation of the therapy or taken at various times during the course of the therapy. In the case of a method or a kit for the screening of a therapeutic treatment to improve the development of follicles with high fertilization potential in a subject, the proper value from a control sample may be the average of the values measured in samples taken from subjects without treatment and from subjects treated with a substance that is to be assayed or from subjects treated with a reference treatment.

Said control sample may be a second aliquot separated from the test sample, wherein it has been preferably introduced a predetermined amount estradiol with isotopic labelling with deuterium (D) or with carbon-13 $^{13}C$, as an internal standard.

In the present invention, the expression "isolated sample from an individual follicular fluid" refers to a sample from an individual follicular fluid among multiple different fluids obtained from subjects under hormonal stimulation treatment. The hormonal stimulation causes the development of multiple follicles, each associated with a single oocyte: each fluid, taken and analyzed separately from the others, gives indications on the quality of the homologous oocyte.

In the present invention, the expression "measuring the amount" is intended as measuring the amount or concentration or level of the respective molecule, preferably semi-quantitative or quantitative. If by comparing the measured amount of the biomarker with the proper value obtained from a control sample of the biomarker, the amount of said biomarker in the test sample isolated from the subject corresponds to a lower value, the subject may present oxidative conditions hindering the development of the dominant follicle associated with oocyte maturation. If by comparing the measured amount of the biomarker with the proper value obtained from a control sample of the biomarker, the amount of said biomarker in the test sample isolated from the subject corresponds to a similar or higher value, in the subject the redox status of the follicle including the oocyte had no (if any) oxidative injury and therefore the follicle have not suffered structural and functional damage.

Measurement of a biomarker can be performed directly or indirectly. Direct measurement refers to the amount or concentration measure of the biomarker, based on a signal obtained directly from the molecule, and which is directly correlated with the number of molecules present in the sample. This signal (which can also be referred to as intensity signal) can be obtained, for example, by measuring an intensity value of a chemical or physical property of the biomarker. Indirect measurements include the measurement obtained from a secondary component and a biological measurement system (e.g. the measurement of cellular responses, ligands, "tags" or enzymatic reaction products).

The term "amount", as used in the description, refers but is not limited to the absolute or relative amount of the biomarker, and any other value or parameter associated with the same or which may result from this. Such values or parameters comprise intensity values of the signal obtained from either physical or chemical properties of the biomarker, obtained by direct measurement, for example, of intensity values in an immunoassay, mass spectroscopy or mass spectrometry or by nuclear magnetic resonance. Additionally, these values or parameters include those obtained by indirect measurement, for example, any of the measurement systems described herein. According to the invention, a predetermined amount of internal standard is used for normalization of the measured value, therefore measured values are normalized with the measurement of the amount of the internal standard.

As used herein, the expression "assessment of the redox state of follicular fluid" indicates the evaluation of the balance between oxidants (plus pro-oxidants) and antioxidants in the follicular fluid, as resulting from oxidation-reduction reactions between electron accepting species and electron donating species, wherein this balance is reflected by the level of structure/function damage of molecule(s) targeted by oxidants. Therefore the redox state of any biological fluid can be assessed by measuring the activity of any molecule depending on the reduction potentials and reducing capacities of the linked redox couples present in the fluid.

## DESCRIPTION OF THE INVENTION

The efficacy of follicular E esterification is directly related to the fact that LCAT is not degraded by oxidative stress. Therefore, the measurement of the follicular level of $E_{EST}$ represents not only a powerful tool to evaluate the correct functioning of the main mechanisms in the export system of E to peripheral tissues, but also an index/biomarker of the follicular redox state. It is worth mentioning that since E and $E_{EST}$ derive almost exclusively from Granulosa cells, the level of these molecules in plasma reflects their production in the follicle. Therefore the assessment of $E_{EST}$ in plasma can be a very useful tool to monitor the redox state in the dominant follicle of women with normal cycles. In particular, in such subjects, a low plasma level of $E_{EST}$ is expected to reflect insufficient LCAT activity in FF and/or oxidative conditions hindering the development of the dominant follicle associated with oocyte maturation. This plasma analysis can be repeated several times during the follicular development of a normal cycle and has the additional advantage of being able to be done on samples collected in a much less invasive way than that represented by follicular puncture and FF aspiration. The expert in the field well knows that circulating $E_{EST}$ are expected to be produced more (if not only) in the follicle, since other steroids (essentially cholesterol) are more abundant substrates for the esterification activity in plasma by the circulating LCAT enzyme. It is known how $E_{EST}$ are transported in plasma and what role they play. So measuring the level of $E_{EST}$ in plasma means knowing how much redox homeostasis is altered within the growing follicle during a normal cycle. However, after hormonal stimulation of superovulation, the plasma level of $E_{EST}$ cannot be associated with the production and esterification of E in a single follicle. To establish whether a low esterification activity in a given preovulatory follicle is correlated with the successful fertilization of the homologous oocyte by functional spermatozoa, an operative pathway in progressive stages can be developed as follows:

1. analysis of the level of $E_{EST}$ both in FF and in homologous plasma (or serum) of subjects who have normal hormonal cycle for follicular development and oocyte maturation;

2. morphological evaluation and finding of positive or unsuccessful performance in fertilization by oocytes, taken from subjects with normal hormonal cycle and analyzed for their level of $E_{EST}$ in FF and homologous plasma (or serum);

3. comparison of oocyte performance in fertilization with

a) the level of $E_{EST}$ in the dominant follicle fluid, and/or

b) the level of $E_{EST}$ in the homologous plasma (or serum);

4. analysis of the level of $E_{EST}$ both in individual FF and in homologous plasma (or serum) taken from subjects who have had hormonal stimulation to induce superovulation, i.e. the development of multiple follicles each containing a mature oocyte;

5. morphological evaluation and finding of positive or unsuccessful performance in fertilization by individual oocytes, taken from subjects who have been stimulated for superovulation and have been analyzed for their levels of $E_{EST}$ in homologous FF;

6. comparison of the performance in fertilization of individual oocytes, taken from subjects stimulated for superovulation, with the level of $E_{EST}$ in homologous FF.

The analysis of the $E_{EST}$ level was carried out with an original procedure. The oocyte morphological evaluation was done with techniques commonly used and currently used in IVF programs. The success of oocyte fertilization has been ascertained in terms of embryonic development in culture up to the blastocyst stage (approximately on the fifth day of development).

In the present invention the $E_{EST}$ analysis was performed in samples from individual fluids to identify, among several fluids from the same subject underwent to hormonal stimulation for superovulation, the fluids with high levels of $E_{EST}$ in order to select homologous oocytes for ART. In fact, it is expected that oocytes from follicles with high $E_{EST}$ have had no (if any) oxidative injury and therefore have not suffered structural and functional damage.

With regard to the $E_{EST}$ analysis in serum/plasma, the present inventors preferably studied plasma collected just at the same time as the fluids. In this way it is possible to ascertain whether the $E_{EST}$ level in plasma (as reflecting the level in the dominant follicle during normal ovulatory cycle) could be used as a marker for monitoring the follicle maturation and get information on the redox state of the follicle including the oocyte. This information is suggested to be useful for predicting the oocyte quality and, in the case of putative low quality of the oocyte, take a decision on whether improving the redox state in subsequent normal ovulatory cycles or to opt for ART.

The method of the invention uses the level of $E_{EST}$ for evaluating follicular maturation and oocyte quality providing information on an important enzymatic activity (i.e. E esterification) that is damaged by a "supraphysiological amount of ROS" o by insufficient antioxidant defences. The present invention is based on the finding that the enzyme esterifying E (i.e. LCAT) is a sensor of oxidative stress, and provides a method wherein the measurement of the activity of this enzyme can be used as a tool to evaluate LCAT functional deficiency, and provides general information on protein damage into the follicle (whether in the fluid or on the oocyte surface) or effects of damaged proteins on the oocyte integrity and fertilization.

The invention will be illustrated by the following figure and non-limitative examples.

**Figure 1.** Diagram of sample preparation for mass spectrometry analysis of $E_{FREE}$ and $E_{TOT}$ levels.

**EXAMPLE 1** - **Analysis of the level of $E_{EST}$ in plasma or FF**

E and $E_{EST}$ in FF and plasma of women with physiological follicular development and oocyte maturation (in normal sexual cycle) and in fluids taken from individual follicles, developed in women stimulated with hormones for superovulation, were analyzed as follows.

Part of each sample of homologous fluid and plasma was used, after appropriate dilutions for routine analysis of E and P according to the CLIA technique, while part (100 $\mu$L) was first supplemented with 2 $\mu$L of 100 $\mu$g/mL of deuterated E (17beta-Estradiol-2,4,16,16,17-D5, here referred to simply as E-d5, provided by Neochema, Bodenheim, Germany), as an internal standard, and then divided into two equal aliquots of 50 $\mu$L. One aliquot, hence called "not saponified" (NS), was supplemented with 0.6 mL of 0.5 M KCl in ethanol. The other aliquot, hence called "saponified" (S) was supplemented with 0.45 mL of 0.5 M KOH in methanol and, after 120 min of incubation at 60 °C, the pH was raised to 6-7 by addition of 13 $\mu$L of acetic acid. Both aliquots, i.e. the NS aliquot (containing E in both free and esterified form) and the S aliquot (containing almost exclusively non-esterified forms of E, as the sum of the originally already free forms plus the originally esterified forms, which was converted by saponification to free forms), were supplemented with 200 $\mu$L of 0.2 M NaCl and then vigorously shaken together with 0.9 mL of $CHCl_3$. After the removal of the aqueous phase, the organic phase was taken: the NS extract contained hydrophobic molecules, including $E_{FREE}$ and $E_{EST}$, while the S extract contained hydrophobic molecules, including $E_{FREE}$ plus E freed by hydrolysis of acyl chains (this sum has been indicated as $E_{TOT}$). The aqueous solution was extracted again with 0.9 mL $CHCl_3$. After this second extraction, the aqueous phase was discarded and the organic phase was taken. The two sequential organic phase extracts for each sample (i.e. from S and NS) were combined and then dried by centrifugation under vacuum or air, at room temperature. Each of the dried sediments was dissolved with 100 $\mu$L of 30 mM $NH_4OH$ in $CH_3OH$/-$H_2O$ (1:1, v/v) and 20 $\mu$L of this solution were injected on the Agilent 6530 Accurate-Mass Q-TOF UPLC/MS system, interfaced with the chromatography column by means of a *nanospray* ion source. Chromatography, carried out in reverse phase with a column of octadecylsilane (C18) conjugated to stationary phase, allowed to fractionate the components of the injected solutions and to isolate E and E-d5 in the same fractions, which were analyzed with the mass spectrometer. In detail, a Zorbax RRHD Eclipse Plus C18 column (2.1 $\times$ 50 mm, 1.8 $\mu$m was used; Agilent, Santa Clara, CA, USA) and elution was carried out at flow rate of 0.150 mL/min with linear gradient from solution A (30 mM $NH_4OH$, 30% $CH_3OH$) to solution B (30 mM $NH_4OH$, 60% $CH_3OH$) in 15 min. For spectrometry, the analysis was done with negative ionization in SIM and MRM mode, monitoring ions at m/z 271.2 (and transition 271.2→145.0) for E and ions at m/z 276.2 (and transition 281.2→150.0) for E-d5. The detected amount of E-d5 was used as an internal standard to normalize the value of $E_{FREE}$ to that of $E_{TOT}$. It has been assumed that the difference between these two values represents the value of esters ($E_{EST} = E_{TOT} -E_{FREE}$), which can be expressed in different ways as, for example, is indicated below:

- "percent of esters in the total population of most esterified free forms", i.e. $E_{EST}\% = E_{EST} \times 100/E_{TOT}$;
- "ratio between free forms and esterified forms", i.e. $E_{FREE} / E_{EST}$ ;
- "ratio between esterified and free forms", i.e. $E_{EST} / E_{FREE}$ ;
- "fraction of esters on the total", i.e. $E_{EST}/E_{TOT}$ .

The method chosen, to describe the experimental data obtained, was the first listed above, namely to indicate the percentage of esters in the total population, i,e, $E_{EST}\% = E_{EST} \times 100/E_{TOT}$. A diagram of sample preparation for mass spectrometry analysis is shown in Figure 1.

The level of $E_{EST}$ was analyzed in 10 subjects with physiological follicular development and in 10 subjects stimulated with hormones to induce the development of multiple follicles (superovulation). In subjects with normal cycle, the level of $E_{EST}$ was found between 7 and 60% in FF and 10 and 70% in plasma, and follicular values were positively correlated with plasma values (r = 0.95). A threshold value separating most positive plasma values of $E_{EST}$ (i.e. those in FF associated with fertilized oocytes) from most negative plasma values (i.e. those in FF associated with unfertilized oocytes) was found in the range of 25 to 35%. The number of FF and oocytes analyzed (4 fertilized and 6 unfertilized) suggests that fertilization capacity is associated with elevated $E_{EST}$ values and the positive correlation between follicular values and plasma values indicates that, in subjects with normal cycle, the level of $E_{EST}$ in plasma reflects the activity of LCAT in the follicle and, therefore, provides information on the follicular redox state without the need to perforate the follicle to aspirate the FF and analyze $E_{EST}$ contained therein.

From the stimulated subjects, 25 FF were collected together with the corresponding oocytes. In these FF, $E_{EST}$ values were found in the range between 4.9 and 83.2% and were examined in the light of the fate of homologous oocytes, i.e. the outcome of in vitro fertilization processes, after 6-4 days of cell culture. In detail, 13 oocytes were fertilized and 8 embryos developed in culture to the appropriate stage for transfer to the uterus. The results clearly indicate that fertilized oocytes were mostly associated with FF with the highest levels of $E_{EST}$, while lower levels were mostly associated with unfertilized oocytes. A threshold value separating most positive $E_{EST}$ values (i.e. FF values associated with fertilized oocytes) from most negative values (i.e. FF values associated with unfertilized oocytes) was found in the range of 20 to 30%. In particular, at the threshold value of 23%, there were 10 true positives, 11 true negatives, 2 false positives and 2 negative falsehoods in the total population of 25 FF examined. These data, when analyzed with a confusion matrix, allowed to establish that the $E_{EST}$ assay to predict the success of an oocyte in IVF has 84% accuracy, 83% sensitivity, 83% precision and 85% specificity.

## DISCUSSION

The main form of application of the present invention is to reveal the amount of $E_{TOT}$ and $E_{FREE}$ in FF of women treated with hormones for superovulation, in order to calculate the level of $E_{EST}$, assuming this level as an index of redox homeostasis in the environment which surrounds the COC and contains signals and factors addressed to oocyte maturation. The detection of oocyte quality indicators, including oocyte success in ART, and the correlation between the follicular level of $E_{EST}$ and the fate of the homologous oocyte are an integral part of this application. Thus, the measurement of the level of $E_{EST}$ in the follicle represents a new criterion for identifying and selecting the oocytes to be processed in ART.

In another application, the level of $E_{EST}$ is calculated in both FF and plasma of women with normal cycles and the positive correlation between the two sources of $E_{TOT}$ and $E_{FREE}$ can be used to verify that the redox state in plasma reflects the redox state in the follicle. Based on the above positive correlation between follicular $E_{EST}$ and oocyte quality, the calculation of the level of $E_{EST}$ in plasma allows to have information on the redox state in the follicle and therefore on the oocyte quality. The link between the plasma level of $E_{EST}$ and the fate of the oocyte is an integral part of this application. Therefore, the measurement of the level of $E_{EST}$ in the plasma of a woman with normal cycle represents a new criterion for predicting the success or failure of the oocyte in physiological or *in vitro* fertilization.

The fact that, in FF, oxidative stress and antioxidant defenses affect LCAT activity in opposite ways presents the opportunity to design treatments to improve oocyte fertilization and embryo development in both hormone-stimulated women and women not stimulated for oocyte maturation. The synthesis and selection of so-called *"lead compounds"* and the development of new drugs for female infertility need biomarkers to evaluate the response to treatment and the activity of LCAT, here referred to the esterification of E in the ovarian follicle or plasma, is proposed as an appropriate marker of the effectiveness of new therapies.

For example, immunoassays such as RIA or CLIA can be used to measure $E_{FREE}$ levels and $E_{TOT}$ to calculate $E_{EST}$. The expert in the field knows well that the levels of $E_{FREE}$ and $E_{TOT}$ can be measured by means of some biochemical or immunochemical techniques, but a technique of choice is based on the use of organic solvents to extract lipids, followed by that of a stationary lipophilic phase to fractionate steroids and other lipids according to their polarity, then by the isolation of steroids by liquid or gas chromatography and, finally, by the detection and quantification of E by mass spectrometry. The expert knows that different organic solvents, different lipophilic phases and different chromatography columns/eluents can be used to isolate and detect E from samples containing $E_{FREE}$ and $E_{TOT}$. Any other procedure or technology for measuring the levels of $E_{FREE}$ and $E_{TOT}$ must be considered an integral part of this invention.

The present invention offers significant advantages on current knowledge and practice, including:

- better accuracy in the identification and selection of oocytes valid for ART;
- better reliability in the identification and selection of oocytes valid for ART;
- non-invasive method for predicting oocyte quality in women with normal hormonal cycles;
- method to evaluate the effectiveness of drugs that increase the antioxidant defenses in the growing follicle;
- method for assessing the effectiveness of drugs that promote oocyte maturation;
- method for evaluating the effectiveness of drugs that treat follicular developmental dysfunctions such as, for example, non-exclusive, polycystic ovary syndrome;
- information on the components needed to assemble oocyte quality analysis kits.

While the foregoing has been set out to illustrate the benefits of this invention, any other modification or use of it must obviously be understood to fall within the broad scope and scope of this invention as defined in the attached claims.

In this invention, the procedure for normalizing the values of samples S and NS, performed to calculate the level of $E_{EST}$, involves the use of E-d5 as an internal standard. Variants of this procedure may be based on the use of different internal standards. As a non-exclusive example, Bromothymol Blue can be used as an internal standard because it efficiently accompanies E during all the steps that process FF and plasma to prepare samples for chromatography and mass spectrometry. In this frame the samples, prior to chromatography and mass spectrometry, should be analyzed for their Bromothymol Blue content. This analysis can be easily done by measuring the internal standard absorbance at 425 or 625 nm, pH 6 or 9 respectively. It must be recognized that each molecule, which can be processed together with FF or plasma without significant losses and without structural alterations through all stages of processing for sample preparation, is in fact an adequate internal standard for the accurate measurement of $E_{EST}$ and $E_{TOT}$.

Another variant of the $E_{EST}$ and $E_{TOT}$ analysis procedure is the use of ethanol (instead of methanol) and ethyl acetate extraction (instead of chloroform) in sample preparation. In this variant, after drying of the extracts and subsequent solubilization of the sediments derived from the S and NS samples, the amount of Bromothymol Blue (or other internal standard) can be measured by spectrophotometry and the levels of $E_{EST}$ and $E_{TOT}$ by RIA or CLIA.

The invention may be expanded into parts, elements and characteristics relating to or indicated in the specifications of the invention, individually or collectively, in each or all combinations of two or more parts, elements or characteristics. It must of course be understood that, while the foregoing has been set forth as an illustrative example of a variant of this invention, any other modification or variant relating thereto, as would be evident to persons experienced in the field, is deemed to fall within the broader scope and scope of this invention, as defined in the attached claims. Aspects of this invention have been described merely by way of example and it must be made clear that modifications and additions can be made in this regard without departing from the scope of the same, as defined in the attached claims.

## REFERENCES

1 - Hart RJ. Physiological aspects of female fertility: role of the environment, modern lifestyle, and genetics. Physiol Rev 2016, 96: 873-909.

2 - Hanson B, Johnstone E, Dorais J, Silver B, Peterson CM, Hotaling J. Female infertility, infertility-associated diagnoses, and comorbidities: a review. J Assist Reprod Genet 2017, 34:167-177.

3 - Harper JC, Aittomäki, Borry P, Cornel MC, de Wert G, Dondorp W, Geraedts J, Gianaroli L, Ketterson K, Liebaers I, Lundin K, Mertes H, Morris M, Pennings G, Sermon K, Spits C, Soini S, van Montfoort APA, Veiga A, Vermeesch JR, Vibville S, Macek M., on behalf of the European Society of Human Reproduction and Embryology and European Society of Human Genetics. Recent developments in genetics and medically-assisted reproduction: from research to clinical applications. Hum Reprod Open 2017, pp 1-20 (doi: 10.1093/hropen/hox015).

4 - Carré J, Gatimel N, Moreau J, Parinaud J, Léandri R. Does air pollution play a role in infertility?: a systematic review. Environmental Health 2017, 16:82 (doi: 10.1186/s12940-017-0291-8).

5 - Sun C, Rong X, Cai Y, Qiu S, Farzaneh M. Mini-review: the FDA-approved prescription drugs that induce ovulation in women with ovulatory problems. Drug Dev Res 2020, 81:815-822.

6 - Fortune JE. Ovarian follicular growth and development in mammals. Biol Reprod 1994, 50:225-232.

7 - Ruder EH, Hartman TJ, Blumberg J, Goldman MB. Oxidative stress and antioxidants: exposure and impact on female fertility. Hum Peprod Update 2008, 14:345-357.

8 - Revelli A, Delle Piane L, Casano S, Molinari E, Massobrio M, Rinaudo P. Follicular fluid content and oocyte quality: from single biochemical markers to metabolomics. Reprod Biol Endocrinol 2009, 7:40-52.

9 - Rodgers RJ, Irving-Rodgers HF. Formation of the ovarian follicular antrum and follicular fluid. Biol Reprod 2010, 82:1021-1029.

10 - Dumesic DA, Meldrum DR, Katz-Jaffe MG, Krisher R, Schoolkraft WB. Oocyte environment: follicular fluid and cumulus cells are critical for oocyte health. Fertil steril 2015, 103:303-316.

11 - De Broi MG, Giorgi VSI, Wang F, Keefe DL, Albertini D, Navarro PA. Influence of follicular fluid and cumulus cells on oocyte quality: clinical implications. J Ass Genet Reprod 2018, 35:735-751.

12 - Agarwal A, Aponte-Mellado A, Premkumar BJ, Shaman A, Gupta S. Effects of oxidative stress on female reproduction: a review. Reprod Biol Endocrinol 2012, 10:49-79.

13 - Freitas C, Neto AC, Matos L, Silva E, Ribeiro A, Silva-Carvalho JL, Almeida H. Follicular fluid redox involvement for ovarian follicle growth. J Ovarian Res 2017, 10:44-53.

14 - Mihalas BP, Redgrove KA, McLaughlin EA, Nixon B. Molecular mechanisms responsible for increased vulnerability af the ageing oocyte to oxidative damage. Oxid Med Cell Longev 2017, 2017:4015874, Epub 2017 Oct 18 (doi: 10.1155/2017/4015874).

15 - Nishihara T, Matsumoto K, Hosoi Y, Morimoto Y. Evaluation of antioxidant status and oxidative stress markers in follicular fluid for human in vitro fertilization outcome. Reprod Mol Biol 2018, 17:481-486.

16 - Lu J, Wang Z, Cao J, Chen Y, Dong Y. A novel and compact review on the role of oxidative stress in female reproduction. Reprod Biol Endocrinol 2018, 16:80-97.

17 - Opuwari SC, Henkel RR. An update on oxidative damage to spermatozoa and oocytes. Biomed Res Int 2016, 2016:9540142, Epub 2016 Jan 28 (doi: 10.1155/2016/9540142).

18 - Luddi A, Capaldo A, Focarelli R, Gori M, Morgante G, Piomboni P, De Leo V. Antioxidants reduce oxidative stress in follicular fluid of aged women undergoing IVF. Reprod Biol Endocrinol 2016, 14:57-63.

19 - Cigliano L, Spagnuolo MS, Dale B, Balestrieri M, Abrescia P. Estradiol esterification in the human preovulatory follicle. Steroids 2001, 66:889-896.

20 - Cigliano I, Balestrieri M, Spagnuolo MS, Dale B, Abrescia P. Lecithin-cholesterol acyltransferase activity during maturation of human preovulatory follicles with different concentration of ascorbate, alpha-tocopherol and nitrotirosine. Reprod Fertil Dev 2002, 14:1-7.

21 - Johnson WJ, Mahlberg FH, Rothblat GH, Phillips M. Cholesterol transport between cells and high-density lipoproteins. Biochim Biophys Acta 1991, 1085:273-298.

22 - Schroeder F, Jefferson JR, Kier AB, Knittel J, Scallen TJ, Wood WG, Hapala I. Membrane cholesterol dynamics: cholesterol domains and kinetic pools. Proc Soc Exp Biol Med 1991, 196:235-252.

23 - Kellner-Weibel G, Jerome WG, Small DM, Warner GJ, Stoltenborg JK, Kearney MA, Corjay MH, Phillips MC, Rothblat GH. Effects of intracellular free cholesterol accumulation on macrophage viability: a model for foam cell death. Arterioscl Thromb Vasc Biol 1998, 18:423-431.

24 - Gore-Langton RE, Armstrong DT. Follicular steroidogenesis and its control. I,: Knobil E and Neill JD editors, The Physiology of Reproduction. Raven Press: New York 1994, 1:629-724.

25 - Greenwald GS, Roy SK. Follicular development. In: Knobil E and Neill JD editors. Raven Press: New York 1994, 1:629-724.

26 - Billig H, Furuta I, Hsueh AJW. Estrogens inhibit and androgens enhance ovarian granulosa apoptosis. Endocrinology 1993, 133:2204-2212.

27 - Erickson GF. The ovary: basic principles and concepts. In: Felig P, Baxter JD, and Frohman LA editors, Endocrinology and Metabolism. McGraw-Hill: New York, 1995:973-1015.

28 - Kaipia A, Hsueh AJW. Regulation of ovarian atresia. Ann Rev Physiol 1997, 59:349-463.

29 - Huet C, Monget P, Pisselet C, Monniaux D. Changes in extracellular matrix components and steroidogenic enzymes during growth and atresia of antral ovarian follicles in the sheep. Biol Reprod 1997, 56:10251103.

30 - Le Goff D. Follicular fluid lipoproteins in the mare: evaluation of HDL transfer from plasma to follicular fluid. Biochim Biophys Acta 1994, 1210:226-232.

31 - Jaspard B, Collet X, Barbaras R, Manent J, Vieu C, Parinaud J, Chap H, Perret B. Biochemical characterization of pre-beta1 high-density lipoprotein from human ovarian follicular fluid: evidence for the presence of a lipid core. Biochemistry 1996, 35:1352-1357.

32 - Ravnik SE, Zarutskie PW, Muller CH. Lipid transfer activity in human follicular fluid: relation to human sperm capacitation. J Androl 1990, 11:216-226.

33 - Lepage N, Miron P, Hemmings R, Roberts KD, Langlais J. Distribution of lysophospholipids and metabolism of platelet-activating factor in human follicular fluid and peritoneal fluids. J Fertil Reprod 1993, 98:349-356.

34 - Shalgi R, Kaicer P, Rimon A, Pinto M, Soferman N. Proteins of human follicular fluid: the blood-follicle barrier. Fertil Steril 1973, 24:429-434.

35 - Simpson ER, Rochelle DB, BR Carr, PC MacDonald. Plasma lipoproteins in follicular fluid of human ovaries. J Clin Endocrinol Metab 1980, 51:1469-1471.

36 - Perret BP, Parinaud J, Ribbes H, Moatti JP, Pontonnier G, Chap H, Douste-Blazy L. Lipoprotein and phospholipid distribution in human follicular fluid. Fertil Steril 1985, 43:405-409.

37 - Volpe A, Coukos G, Uccelli E, Droghini F. Adamo R, Artini PG. Follicular fluid lipoproteins in preovulatory period and their relationship with follicular maturation and progesterone production by human granulosa-luteal cells in vivo and in vitro. J Endocrinol Invest 1991, 14:737-742.

38 - Azhar S, Tsai L, Medicarla S, Chandrasekher Y, Judge L, Reaven E. Human granulosa cells use high density lipoprotein cholesterol for steroidogenesis. J Clin Endocrinol Metab 1998, 83:983-991.

39 - Hochberg RB. Biological esterification of steroids. Endocrinol Rev 1998, 19:331-348.

40 - Larner JM, Shackleton CH, Roitman E, Schwartz PE, Hochberg RB. Measurement of estradiol-17-fatty acid esters in human tissues. J Clin Endocrinol Metab 1992, 75:195-200.

41 - Larner JM, Hochberg RB. The clearance and metabolism of estradiol and estradiol-17-esters in the rat. Endocrinology 1985, 117:1209-1214.

42 - Ng ST, Chang TH, Wu TC. Prediction of the rates of fertilization, cleavage, and pregnancy success by cumulus-coronal morphology in an in vitro fertilization program. Fertil Steril 1999, 72:412-417.

43 - Rattanachaiyanont M, Leader A, Leveille MC. Lack of correlation between oocyte-corona-cumulus complex morphology and nuclear maturity of oocytes collected in stimulated cycles for intracytoplasmic sperm injection. Fertil Steril 1999, 71:937-940.

44 - Lee KS, Joo BS, Na YJ, Yoon MS, Choi OH, Kim WW. Cumulus cells apoptosis as an indicator to predict the quality of oocytes and the outcome of IVF-ET. J Assist Reprod Genet 2001, 18:490-498.

45 - Moffatt O, Drury S, Tomlinson M, Afnan M, Sakkas D. The apoptotic profile of human cumulus cells changes with patient age and after exposure to sperm but not in relation to oocyte maturity. Fertil Steril 2002, 77: 1006-1011.

46 - Babayan A, Neuer A, Dieterle S, Bongiovanni AM and Witkin SS. Hyaluronan in follicular fluid and embryo implantation following in vitro fertilization and embryo transfer. J Assist Reprod Genet 2008, 25:473-476.

47 - Pabuccu R, Kaya C, Cağlar GS, Oztas E and Satiroglu H. Follicular-fluid anti-Mullerian hormone concentrations are predictive of assisted reproduction outcome in PCOS patients. Reprod Biomed Online 2009, 19:631-637.

48 - Ozkan S, Jindal S, Greenseid K, Shu J, Zeitlian G, Hickmon C and Pal L. Replete vitamin D stores predict reproductive success following in vitro fertilization. Fertil Steril 2010; 94: 1314-1319.

49 - Wallace M, Cottell E, Gibney MJ, McAuliffe FM, Wingfield M and Brennan L. An investigation into the relationship between the metabolic profile of follicular fluid, oocyte developmental potential, and implantation outcome. Fertil Steril 2012, 97:1078-1084, e1078.

50 - Bianchi L, Gagliardi A, Campanella G, Landi C, Capaldo A, Carleo A, Armini A, De Leo V, Piomboni P, Focarelli R and Bini L. A methodological and functional proteomic approach of human follicular fluid en route for oocyte quality evaluation. J Proteomics 2013, 90:61-76.

51 - Lédée N, Gridelet V, Ravet S, Jouan C, Gaspard O, Wenders F, Thonon F, Hincourt N, Dubois M and Foidart JM. Impact of follicular G-CSF quantification on subsequent embryo transfer decisions: a proof of concept study. Hum Reprod 2013, 28:406-413.

52 - Xia L, Zhao X, Sun Y, Hong Y, Gao Y, Hu S. Metabolomic profiling of human follicular fluid from patients with repeated failure of in vitro fertilization using gas chromatography/mass spectrometry. Int J Clin Paxol XP 2014, 7:7220-7229.

53 - Corn CM, Hauser-Kronberger C, Moser M, Tews G, Ebner T. Predictive value of cumulus cell apoptosis with regard to blastocyst development of corresponding gametes. Fertil Steril 2005, 84:627-633.

54 - Scott L, Alvero R, Leondires M, Miller B. The morphology of human pronuclear embryos is positively related to blastocyst development and implantation. Hum Reprod 2000, 15: 2394-2403.

55 - De Placido G, Wilding M, Strina I, Alviggi E, Mollo A, Vricchio M, Tolino A, Schiattarella C, Dale B. High outcome predictability After IVF using a combined score for zygote and embryo morphology and growth rate., Human Reproduction 2002, 17:2402-2409.

56 - Balaban B, Urman B. Effect of oocyte morphology on embryo development and implantation. Reprod Biomed Online 2006, 12, 608-615.

57 - Wilding M, Di Matteo L, D'Andretti S, Montanaro N, Capobianco C, Dale B. An oocyte score for use in assisted reproduction . J Assist Reprod Genet 2007, 24:350-358.

58 - La Sala GB, Nicoli A, Villani MT, Di Girolamo R, Capodanno F, Blickstein I. The effect of selecting oocytes for insemination and transferring all resultant embryos without selection on outcomes of assisted reproduction. Fertil Steril 2009, 91:96-100.

59 - Fenwick J, Platteau P, Murdoch AP, Herbert M. Time from insemination to first cleavage predicts development competence of human preimplantation embryos in vitro. Hum Reprod 2002, 17:407-412.
60 - Vihma V, Adlercreutz H, Tiitinen A, Kiuru P, Wähälä K, Tikkanen M. Quantitative Determination of Estradiol Fatty Acid Esters in Human Pregnancy Serum and Ovarian Follicular Fluid. Clin Chem 2001, 47:1256-1262.
61 - Chen F, Spiesseng C, D'Hooghe T, Peeraer K, Carpentier S. Follicular fluid biomarkers for human in vitro fertilization outcome: Proof of principle. Proteome Science 2016, 14:1-11.

**Claims**

1. An *in vitro* method for the evaluation of oocytes quality and/or of the response to treatments to improve the development of follicles with high fertilization potential in a subject, said method comprising the step of measuring the amount of esterified estradiol ($E_{EST}$) in an isolated sample of plasma or follicular fluid from said subject and comparing the same with a $E_{EST}$ control value indicative of subjects with oocytes with good fertilization potential.

2. The method according to claim 1 comprising the following steps:

   a. measuring in the isolated sample of plasma or follicular fluid the amounts of total estradiol ($E_{TOT}$) and of non-esterified estradiol ($E_{FREE}$);
   b. calculating the amount of $E_{EST}$ according to the following formula: $E_{EST} = E_{TOT} - E_{FREE}$
   c. comparing the obtained value as in step b) with the $E_{EST}$ control value.

3. The method according to claim 1 or 2 comprising the steps of:

   a. introducing a predetermined amount of internal standard in the isolated sample of plasma or follicular fluid;
   b. separating said sample into a first and a second aliquot;
   c. treating said first aliquot by means of saponification;
   d. treating both aliquots with organic extraction and taking the organic extract;
   e. measuring the amount of $E_{FREE}$ in the organic extracts of the first and second aliquot and using the internal standard amounts for normalization of the obtained value;
   f. calculating the amount of $E_{EST}$ according to the formula: $E_{EST} = E_{TOT} - E_{FREE}$, wherein the $E_{TOT}$ value corresponds to the amount of $E_{FREE}$ measured in the first aliquot and the $E_{FREE}$ value corresponds to the amount of $E_{FREE}$ measured in the second aliquot;
   g. comparing the obtained $E_{EST}$ value with the

$E_{EST}$ control value.

4. The method according to claim 3 wherein the internal standard is estradiol with isotopic labelling with deuterium (D) or with carbon-13 $^{13}$C, preferably 17β-Estradiol-2,4,16,16,17-D$_5$, 17β-Estradiol-16,16,17-D$_3$, 17β-Estradiol-2,4,16,16,17-D$_2$, 17β-Estradiol-2,3,4-$^{13}$C$_3$.

5. The method according to any of previous claims, wherein the amount of $E_{EST}$ is expressed as percentage $E_{EST}/E_{TOT} \times 100$.

6. The method according to any of previous claims wherein the isolated sample of individual follicular fluid is obtained from subjects with multiple follicles developed under hormonal stimulation treatment.

7. The method according to claims 5 and 6 wherein the $E_{EST}$ control value is comprised between 20 and 30% and wherein the $E_{EST}$ value measured in the biological sample of individual follicular fluid greater than said control value is indicative of homologous oocyte with good fertilization potential.

8. The method according to any one of claims 1 - 5 wherein the isolated sample is an isolated plasma sample obtained from subjects with a normal ovulatory cycle.

9. The method according to claims 1 and 8 wherein the $E_{EST}$ control value is comprised between 25 and 35% and wherein the $E_{EST}$ value measured in the isolated plasma sample greater than said control value is indicative of oocytes with good fertilization potential.

10. Use of a kit comprising the means for measuring the amount of the biomarker $E_{EST}$ and, optionally, control means, for working the method of any one of claims 1 to 9.

**Patentansprüche**

1. *In-vitro*-Verfahren zur Beurteilung der Qualität von Eizellen und/oder des Ansprechens auf Behandlungen zur Verbesserung der Entwicklung von Follikeln mit hohem Befruchtungspotenzial bei einem Individuum, wobei das Verfahren den Schritt des Messens der Menge an verestertem Estradiol ($E_{EST}$) in einer isolierten Plasma- oder Follikelflüssigkeitsprobe des Individuums und deren Vergleich mit einem $E_{EST}$-Kontrollwert umfasst, der für Individuen mit Eizellen mit gutem Befruchtungspotenzial indikativ ist.

2. Verfahren nach Anspruch 1 umfassend die folgenden Schritte:

a. Messen der Mengen an Gesamtestradiol ($E_{GESAMT}$) und an nicht verestertem Östradiol ($E_{FREI}$) in der isolierten Plasma- oder Follikelflüssigkeitsprobe;
b. Berechnen der Menge von $E_{EST}$ nach folgender Formel:

$$E_{EST} = E_{GESAMT} - E_{FREI}$$

c. Vergleichen des erhaltenen Wertes wie in Schritt b) mit dem $E_{EST}$-Kontrollwert.

3. Verfahren nach Anspruch 1 oder 2 umfassend die folgenden Schritte:

a. Einbringen einer vorab festgelegten Menge eines internen Standards in die isolierte Plasma- oder Follikelflüssigkeitsprobe;
b. Aufteilen der Probe in ein erstes und ein zweites Aliquot;
c. Behandeln des ersten Aliquots mittels Verseifung;
d. Behandeln beider Aliquots mit organischer Extraktion und Entnahme des organischen Extrakts;
e. Messen der Menge an $E_{FREI}$ in den organischen Extrakten des ersten und des zweiten Aliquots und Verwenden der Mengen des internen Standards zum Normalisieren des erhaltenen Wertes;
f. Berechnen der $E_{EST}$-Menge nach der Formel: $E_{EST} = E_{GESAMT} - E_{FREI}$, wobei der $E_{GESAMT}$-Wert der $E_{FREI}$-Menge entspricht, die in dem ersten Aliquot gemessen wurde, und der $E_{FREI}$-Wert der $E_{FREI}$-Menge entspricht, die in dem zweiten Aliquot gemessen wurde;
g. Vergleichen des erhaltenen $E_{EST}$-Werts mit dem $E_{EST}$-Kontrollwert.

4. Verfahren nach Anspruch 3, wobei es sich bei dem internen Standard um Estradiol mit Isotopenmarkierung mit Deuterium (D) oder mit Kohlenstoff-13 $^{13}$C handelt, vorzugsweise um 17β-Estradiol-2,4,16,16,17-D$_5$, 17β-Estradiol-16,16,17-D$_3$, 17β-Estradiol-2,4,16,16,17-D$_2$, 17β-Estradiol-2,3,4-$^{13}$C$_3$.

5. Verfahren nach einem der vorhergehenden Ansprüche, wobei die Menge von $E_{EST}$ als Prozentsatz $E_{EST}/E_{GESAMT} \times 100$ ausgedrückt wird.

6. Verfahren nach einem der vorhergehenden Ansprüche, wobei die isolierte Probe einer einzelnen Follikelflüssigkeit von Individuen mit mehreren Follikeln gewonnen wird, die sich unter einer hormonellen Stimulationsbehandlung entwickelt haben.

7. Verfahren nach den Ansprüchen 5 und 6, wobei der $E_{EST}$-Kontrollwert zwischen 20 und 30 % liegt und wobei der in der biologischen Probe einer einzelnen Follikelflüssigkeit gemessene $E_{EST}$-Wert, der größer als der Kontrollwert ist, für eine homologe Eizelle mit gutem Befruchtungspotenzial indikativ ist.

8. Verfahren nach einem der Ansprüche 1 bis 5, wobei es sich bei der isolierten Probe um eine isolierte Plasmaprobe handelt, die von Individuen mit einem normalen Ovulationszyklus erhalten wurde.

9. Verfahren nach den Ansprüchen 1 und 8, wobei der $E_{EST}$-Kontrollwert zwischen 25 und 35 % liegt und wobei der in der isolierten Plasmaprobe gemessene $E_{EST}$-Wert, der größer als der Kontrollwert ist, für Eizellen mit gutem Befruchtungspotenzial indikativ ist.

10. Verwendung eines Kits umfassend die Mittel zum Messen der Menge des Biomarkers $E_{EST}$ und wahlweise Kontrollmittel zum Durchführen des Verfahrens nach einem der Ansprüche 1 bis 9.

**Revendications**

1. Procédé *in vitro* pour l'évaluation de la qualité d'ovocytes et/ou de la réponse aux traitements pour améliorer le développement de follicules avec un potentiel de fécondation élevé chez un sujet, ledit procédé comprenant l'étape de mesure de la quantité d'estradiol estérifié ($E_{EST}$) dans un échantillon isolé de plasma ou de fluide folliculaire dudit sujet et comparer le même avec une valeur témoin d'$E_{EST}$ indicatrice de sujets avec un bon potentiel de fécondation.

2. Procédé selon la revendication 1 comprenant les étapes suivantes consistant à :

a. mesurer dans l'échantillon isolé de plasma ou le fluide folliculaire les quantités d'estradiol total ($E_{TOT}$) et d'estradiol non estérifié ($E_{LIBRE}$) ;
b. calculer la quantité d'$E_{EST}$ selon la formule suivante : $E_{EST} = E_{TOT} - E_{LIBRE}$
c. comparer la valeur obtenue comme dans l'étape b) avec la valeur témoin d'$E_{EST}$.

3. Procédé selon la revendication 1 ou 2 comprenant les étapes consistant à :

a. introduire une quantité prédéterminée de standard interne dans l'échantillon isolé de plasma ou le fluide folliculaire ;
b. séparer ledit échantillon en une première et une seconde aliquotes ;

c. traiter ladite première aliquote au moyen de saponification ;

d. traiter les deux aliquotes avec une extraction organique et prendre l'extrait organique ;

e. mesurer la quantité d'$E_{LIBRE}$ dans les extraits organiques de la première et de la seconde aliquotes et utiliser les quantités standard internes pour la normalisation de la valeur obtenue ;

f. calculer la quantité d'$E_{EST}$ selon la formule : $E_{EST} = E_{TOT} - E_{LIBRE}$, la valeur d'$E_{TOT}$ correspondant à la quantité d'$E_{LIBRE}$ mesurée dans la première aliquote et la valeur d'$E_{LIBRE}$ correspondant à la quantité d'$E_{LIBRE}$ mesurée dans la seconde aliquote ;

g. comparer la valeur d'$E_{EST}$ obtenue avec la valeur témoin d'$E_{EST}$.

4. Procédé selon la revendication 3 dans lequel le standard interne est l'estradiol avec un marquage isotopique avec du deutérium (D) ou avec du carbone 13 $^{13}C$, de préférence le 17β-estradiol-2,4,16,16,17-$D_5$, le 17β-estradiol-16,16,17-$D_3$, le 17β-estradiol-2,4,16,16,17-$D_2$, le 17β-estradiol-2,3,4-$^{13}C_3$.

5. Procédé selon l'une quelconque des revendications précédentes, dans lequel la quantité d $E_{EST}$ est exprimée comme pourcentage $E_{EST}/E_{TOT}$ x 100.

6. Procédé selon l'une quelconque des revendications précédentes, dans lequel l'échantillon isolé de fluide folliculaire individuel est obtenu de sujets avec de multiples follicules développés sous traitement de stimulation hormonale.

7. Procédé selon les revendications 5 et 6 dans lequel la valeur témoin d'$E_{EST}$ est comprise entre 20 et 30 % et la valeur d'$E_{EST}$ mesurée dans l'échantillon biologique de fluide folliculaire individuel supérieure à ladite valeur témoin étant indicatrice d'ovocytes homologues avec un bon potentiel de fécondation.

8. Procédé selon l'une quelconque des revendications 1 à 5 dans lequel l'échantillon isolé est un échantillon de plasma isolé obtenu de sujets avec un cycle ovulatoire normal.

9. Procédé selon les revendications 1 et 8 dans lequel la valeur témoin d'$E_{EST}$ est comprise entre 25 et 35 % et la valeur d'$E_{EST}$ mesurée dans l'échantillon de plasma isolé supérieure à ladite valeur témoin étant indicatrice d'ovocytes avec un bon potentiel de fécondation.

10. Utilisation d'un kit comprenant les moyens pour mesurer la quantité du biomarqueur $E_{EST}$ et éventuellement, les moyens de contrôle, pour travailler le procédé selon l'une quelconque des revendications 1 à 9.

EP 4 345 456 B1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HART RJ**. Physiological aspects of female fertility: role of the environment, modern lifestyle, and genetics. *Physiol Rev*, 2016, vol. 96, 873-909
- **HANSON B** ; **JOHNSTONE E** ; **DORAIS J** ; **SILVER B** ; **PETERSON CM** ; **HOTALING J.** Female infertility, infertility-associated diagnoses, and comorbidities: a review.. *J Assist Reprod Genet*, 2017, vol. 34, 167-177
- **HARPER JC** ; **AITTOMÄKI, BORRY P** ; **CORNEL MC** ; **DE WERT G** ; **DONDORP W** ; **GERAEDTS J** ; **GIANAROLI L** ; **KETTERSON K** ; **LIEBAERS I** ; **LUNDIN K**. Recent developments in genetics and medically-assisted reproduction: from research to clinical applications. *Hum Reprod Open*, 2017, 1-20
- **CARRÉ J** ; **GATIMEL N** ; **MOREAU J** ; **PARINAUD J** ; **LÉANDRI R**. Does air pollution play a role in infertility?: a systematic review. *Environmental Health*, 2017, vol. 16, 82
- **SUN C** ; **RONG X** ; **CAI Y** ; **QIU S** ; **FARZANEH M**. Mini-review: the FDA-approved prescription drugs that induce ovulation in women with ovulatory problems. *Drug Dev Res*, 2020, vol. 81, 815-822
- **FORTUNE JE**. Ovarian follicular growth and development in mammals. *Biol Reprod*, 1994, vol. 50, 225-232
- **RUDER EH** ; **HARTMAN TJ** ; **BLUMBERG J** ; **GOLDMAN MB**. Oxidative stress and antioxidants: exposure and impact on female fertility. *Hum Peprod Update*, 2008, vol. 14, 345-357
- **REVELLI A** ; **DELLE PIANE L** ; **CASANO S** ; **MOLINARI E** ; **MASSOBRIO M** ; **RINAUDO P**. Follicular fluid content and oocyte quality: from single biochemical markers to metabolomics. *Reprod Biol Endocrinol*, 2009, vol. 7, 40-52
- **RODGERS RJ** ; **IRVING-RODGERS HF**. Formation of the ovarian follicular antrum and follicular fluid. *Biol Reprod*, 2010, vol. 82, 1021-1029
- **DUMESIC DA** ; **MELDRUM DR** ; **KATZ-JAFFE MG** ; **KRISHER R** ; **SCHOOLKRAFT WB**. Oocyte environment: follicular fluid and cumulus cells are critical for oocyte health. *Fertil steril*, 2015, vol. 103, 303-316
- **DE BROI MG** ; **GIORGI VSI** ; **WANG F** ; **KEEFE DL** ; **ALBERTINI D** ; **NAVARRO PA**. Influence of follicular fluid and cumulus cells on oocyte quality: clinical implications.. *J Ass Genet Reprod*, 2018, vol. 35, 735-751

- **AGARWAL A** ; **APONTE-MELLADO A** ; **PREMKUMAR BJ** ; **SHAMAN A** ; **GUPTA S**. Effects of oxidative stress on female reproduction: a review. *Reprod Biol Endocrinol*, 2012, vol. 10, 49-79
- **FREITAS C** ; **NETO AC** ; **MATOS L** ; **SILVA E** ; **RIBEIRO A** ; **SILVA-CARVALHO JL** ; **ALMEIDA H**. Follicular fluid redox involvement for ovarian follicle growth.. *J Ovarian Res*, 2017, vol. 10, 44-53
- **MIHALAS BP** ; **REDGROVE KA** ; **MCLAUGHLIN EA** ; **NIXON B**. Molecular mechanisms responsible for increased vulnerability af the ageing oocyte to oxidative damage. *Oxid Med Cell Longev*, 18 October 2017, vol. 2017, 4015874
- **NISHIHARA T** ; **MATSUMOTO K** ; **HOSOI Y** ; **MORIMOTO Y**. Evaluation of antioxidant status and oxidative stress markers in follicular fluid for human in vitro fertilization outcome. *Reprod Mol Biol*, 2018, vol. 17, 481-486
- **LU J** ; **WANG Z** ; **CAO J** ; **CHEN Y** ; **DONG Y**. A novel and compact review on the role of oxidative stress in female reproduction. *Reprod Biol Endocrinol*, 2018, vol. 16, 80-97
- **OPUWARI SC** ; **HENKEL RR**. An update on oxidative damage to spermatozoa and oocytes. *Biomed Res Int*, 28 January 2016, vol. 2016, 9540142
- **LUDDI A** ; **CAPALDO A** ; **FOCARELLI R** ; **GORI M** ; **MORGANTE G** ; **PIOMBONI P** ; **DE LEO V**. Antioxidants reduce oxidative stress in follicular fluid of aged women undergoing IVF. *Reprod Biol Endocrinol*, 2016, vol. 14, 57-63
- **CIGLIANO L** ; **SPAGNUOLO MS** ; **DALE B** ; **BALESTRIERI M** ; **ABRESCIA P**. Estradiol esterification in the human preovulatory follicle.. *Steroids*, 2001, vol. 66, 889-896
- **CIGLIANO L** ; **BALESTRIERI M** ; **SPAGNUOLO MS** ; **DALE B** ; **ABRESCIA P**. Lecithin-cholesterol acyltransferase activity during maturation of human preovulatory follicles with different concentration of ascorbate, alpha-tocopherol and nitrotirosine. *Reprod Fertil Dev*, 2002, vol. 14, 1-7
- **JOHNSON WJ** ; **MAHLBERG FH** ; **ROTHBLAT GH** ; **PHILLIPS M**. Cholesterol transport between cells and high-density lipoproteins.. *Biochim Biophys Acta*, 1991, vol. 1085, 273-298
- **SCHROEDER F** ; **JEFFERSON JR** ; **KIER AB** ; **KNITTEL J** ; **SCALLEN TJ** ; **WOOD WG** ; **HAPALA I**. Membrane cholesterol dynamics: cholesterol domains and kinetic pools. *Proc Soc Exp Biol Med*, 1991, vol. 196, 235-252

- **KELLNER-WEIBEL G** ; **JEROME WG** ; **SMALL DM** ; **WARNER GJ** ; **STOLTENBORG JK** ; **KEARNEY MA** ; **CORJAY MH** ; **PHILLIPS MC** ; **ROTHBLAT GH**. Effects of intracellular free cholesterol accumulation on macrophage viability: a model for foam cell death. *Arterioscl Thromb Vasc Biol*, 1998, vol. 18, 423-431
- Follicular steroidogenesis and its control. I. **GORE-LANGTON RE** ; **ARMSTRONG DT**. The Physiology of Reproduction. Raven Press, 1994, vol. 1, 629-724
- **GREENWALD GS** ; **ROY SK**. Follicular development. Raven Press, 1994, vol. 1, 629-724
- **BILLIG H** ; **FURUTA I** ; **HSUEH AJW**. Estrogens inhibit and androgens enhance ovarian granulosa apoptosis. *Endocrinology*, 1993, vol. 133, 2204-2212
- The ovary: basic principles and concepts. **ERICK-SON GF**. Endocrinology and Metabolism. McGraw-Hill, 1995, 973-1015
- **KAIPIA A** ; **HSUEH AJW**. Regulation of ovarian atresia. *Ann Rev Physiol*, 1997, vol. 59
- **HUET C** ; **MONGET P** ; **PISSELET C** ; **MONNIAUX D**. Changes in extracellular matrix components and steroidogenic enzymes during growth and atresia of antral ovarian follicles in the sheep. *Biol Reprod*, 1997, vol. 56, 10251103
- **LE GOFF D**. Follicular fluid lipoproteins in the mare: evaluation of HDL transfer from plasma to follicular fluid.. *Biochim Biophys Acta*, 1994, vol. 1210, 226-232
- **JASPARD B** ; **COLLET X** ; **BARBARAS R** ; **MANENT J** ; **VIEU C** ; **PARINAUD J** ; **CHAP H** ; **PERRET B.** Biochemical characterization of pre-beta1 high-density lipoprotein from human ovarian follicular fluid: evidence for the presence of a lipid core.. *Biochemistry*, 1996, vol. 35, 1352-1357
- **RAVNIK SE** ; **ZARUTSKIE PW** ; **MULLER CH**. Lipid transfer activity in human follicular fluid: relation to human sperm capacitation.. *J Androl*, 1990, vol. 11, 216-226
- **LEPAGE N** ; **MIRON P** ; **HEMMINGS R** ; **ROBERTS KD** ; **LANGLAIS J.** Distribution of lysophospholipids and metabolism of platelet-activating factor in human follicular fluid and peritoneal fluids.. *J Fertil Reprod*, 1993, vol. 98, 349-356
- **SHALGI R** ; **KAICER P** ; **RIMON A** ; **PINTO M** ; **SOFERMAN N**. Proteins of human follicular fluid: the blood-follicle barrier.. *Fertil Steril*, 1973, vol. 24, 429-434
- **SIMPSON ER** ; **ROCHELLE DB** ; **BR CARR** ; **PC MACDONALD**. Plasma lipoproteins in follicular fluid of human ovaries.. *J Clin Endocrinol Metab*, 1980, vol. 51, 1469-1471
- **PERRET BP** ; **PARINAUD J** ; **RIBBES H** ; **MOATTI JP** ; **PONTONNIER G** ; **CHAP H** ; **DOUSTE-BLAZY L**. Lipoprotein and phospholipid distribution in human follicular fluid. *Fertil Steril*, 1985, vol. 43, 405-409
- **VOLPE A** ; **COUKOS G** ; **UCCELLI E** ; **DROGHINI F** ; **ADAMO R** ; **ARTINI PG**. Follicular fluid lipoproteins in preovulatory period and their relationship with follicular maturation and progesterone production by human granulosa-luteal cells in vivo and in vitro. *J Endocrinol Invest*, 1991, vol. 14, 737-742
- **AZHAR S** ; **TSAI L** ; **MEDICARLA S** ; **CHANDRA-SEKHER Y** ; **JUDGE L** ; **REAVEN E**. Human granulosa cells use high density lipoprotein cholesterol for steroidogenesis.. *J Clin Endocrinol Metab*, 1998, vol. 83, 983-991
- **HOCHBERG RB**. Biological esterification of steroids. *Endocrinol Rev*, 1998, vol. 19, 331-348
- **LARNER JM** ; **SHACKLETON CH** ; **ROITMAN E** ; **SCHWARTZ PE** ; **HOCHBERG RB**. Measurement of estradiol-17-fatty acid esters in human tissues.. *J Clin Endocrinol Metab*, 1992, vol. 75, 195-200
- **LARNER JM** ; **HOCHBERG RB**. The clearance and metabolism of estradiol and estradiol-17-esters in the rat. *Endocrinology*, 1985, vol. 117, 1209-1214
- **NG ST** ; **CHANG TH** ; **WU TC**. Prediction of the rates of fertilization, cleavage, and pregnancy success by cumulus-coronal morphology in an in vitro fertilization program. *Fertil Steril*, 1999, vol. 72, 412-417
- **RATTANACHAIYANONT M** ; **LEADER A** ; **LE-VEILLE MC**. Lack of correlation between oocyte-corona-cumulus complex morphology and nuclear maturity of oocytes collected in stimulated cycles for intracytoplasmic sperm injection.. *Fertil Steril*, 1999, vol. 71, 937-940
- **LEE KS** ; **JOO BS** ; **NA YJ** ; **YOON MS** ; **CHOI OH** ; **KIM WW**. Cumulus cells apoptosis as an indicator to predict the quality of oocytes and the outcome of IVF-ET.. *J Assist Reprod Genet*, 2001, vol. 18, 490-498
- **MOFFATT O** ; **DRURY S** ; **TOMLINSON M** ; **AFNAN M** ; **SAKKAS D**. The apoptotic profile of human cumulus cells changes with patient age and after exposure to sperm but not in relation to oocyte maturity. *Fertil Steril*, 2002, vol. 77, 1006-1011
- **BABAYAN A** ; **NEUER A** ; **DIETERLE S** ; **BONGIO-VANNI AM** ; **WITKIN SS**. Hyaluronan in follicular fluid and embryo implantation following in vitro fertilization and embryo transfer.. *J Assist Reprod Genet*, 2008, vol. 25, 473-476
- **PABUCCU R** ; **KAYA C** ; **CAĞLAR GS** ; **OZTAS E** ; **SATIROGLU H**. Follicular-fluid anti-Mullerian hormone concentrations are predictive of assisted reproduction outcome in PCOS patients. *Reprod Biomed Online*, 2009, vol. 19, 631-637

- **OZKAN S** ; **JINDAL S** ; **GREENSEID K** ; **SHU J** ; **ZEITLIAN G** ; **HICKMON C** ; **PAL L**. Replete vitamin D stores predict reproductive success following in vitro fertilization. *Fertil Steril*, 2010, vol. 94, 1314-1319
- **WALLACE M** ; **COTTELL E** ; **GIBNEY MJ** ; **MCAULIFFE FM** ; **WINGFIELD M** ; **BRENNAN L**. An investigation into the relationship between the metabolic profile of follicular fluid, oocyte developmental potential, and implantation outcome. *Fertil Steril*, 2012, vol. 97, 1078-1084, 1078
- **BIANCHI L** ; **GAGLIARDI A** ; **CAMPANELLA G** ; **LANDI C** ; **CAPALDO A** ; **CARLEO A** ; **ARMINI A** ; **DE LEO V** ; **PIOMBONI P** ; **FOCARELLI R**. A methodological and functional proteomic approach of human follicular fluid en route for oocyte quality evaluation.. *J Proteomics*, 2013, vol. 90, 61-76
- **LÉDÉE N** ; **GRIDELET V** ; **RAVET S** ; **JOUAN C** ; **GASPARD O** ; **WENDERS F** ; **THONON F** ; **HINCOURT N** ; **DUBOIS M** ; **FOIDART JM**. Impact of follicular G-CSF quantification on subsequent embryo transfer decisions: a proof of concept study. *Hum Reprod*, 2013, vol. 28, 406-413
- **XIA L** ; **ZHAO X** ; **SUN Y** ; **HONG Y** ; **GAO Y** ; **HU S**. Metabolomic profiling of human follicular fluid from patients with repeated failure of in vitro fertilization using gas chromatography/mass spectrometry. *Int J Clin Paxol XP*, 2014, vol. 7, 7220-7229
- **CORN CM** ; **HAUSER-KRONBERGER C** ; **MOSER M** ; **TEWS G** ; **EBNER T**. Predictive value of cumulus cell apoptosis with regard to blastocyst development of corresponding gametes. *Fertil Steril*, 2005, vol. 84, 627-633
- **SCOTT L** ; **ALVERO R** ; **LEONDIRES M** ; **MILLER B**. The morphology of human pronuclear embryos is positively related to blastocyst development and implantation. *Hum Reprod*, 2000, vol. 15, 2394-2403
- **DE PLACIDO G** ; **WILDING M** ; **STRINA I** ; **ALVIGGI E** ; **MOLLO A** ; **VRICCHIO M** ; **TOLINO A** ; **SCHIATTARELLA C** ; **DALE B.** High outcome predictability After IVF using a combined score for zygote and embryo morphology and growth rate.. *Human Reproduction*, 2002, vol. 17, 2402-2409
- **BALABAN B** ; **URMAN B.** Effect of oocyte morphology on embryo development and implantation. *Reprod Biomed Online*, 2006, vol. 12, 608-615
- **WILDING M** ; **DI MATTEO L** ; **D'ANDRETTI S** ; **MONTANARO N** ; **CAPOBIANCO C** ; **DALE B.** An oocyte score for use in assisted reproduction .. *J Assist Reprod Genet*, 2007, vol. 24, 350-358
- **LA SALA GB** ; **NICOLI A** ; **VILLANI MT** ; **DI GIROLAMO R** ; **CAPODANNO F** ; **BLICKSTEIN I**. The effect of selecting oocytes for insemination and transferring all resultant embryos without selection on outcomes of assisted reproduction. *Fertil Steril*, 2009, vol. 91, 96-100
- **FENWICK J** ; **PLATTEAU P** ; **MURDOCH AP** ; **HERBERT M**. Time from insemination to first cleavage predicts development competence of human preimplantation embryos in vitro. *Hum Reprod*, 2002, vol. 17, 407-412
- **VIHMA V** ; **ADLERCREUTZ H** ; **TIITINEN A** ; **KIURU P** ; **WÄHÄLÄ K** ; **TIKKANEN M**. Quantitative Determination of Estradiol Fatty Acid Esters in Human Pregnancy Serum and Ovarian Follicular Fluid.. *Clin Chem*, 2001, vol. 47, 1256-1262
- **CHEN F** ; **SPIESSENG C** ; **D'HOOGHE T** ; **PEER-AER K** ; **CARPENTIER S**. Follicular fluid biomarkers for human in vitro fertilization outcome: Proof of principle. *Proteome Science*, 2016, vol. 14, 1-11